(19) **Europäisches Patentamt / European Patent Office / Office européen des brevets**

(11) **EP 3 331 353 B1**

(12) **FASCICULE DE BREVET EUROPEEN**

(45) Date de publication et mention
de la délivrance du brevet:
**04.12.2019 Bulletin 2019/49**

(21) Numéro de dépôt: **16750405.9**

(22) Date de dépôt: **22.07.2016**

(51) Int Cl.:
**A01K 1/03** (2006.01)     **A61B 5/11** (2006.01)

(86) Numéro de dépôt international:
**PCT/EP2016/067520**

(87) Numéro de publication internationale:
**WO 2017/017010 (02.02.2017 Gazette 2017/05)**

(54) **DISPOSITIF INSTRUMENTALISE POUR LA CARACTERISATION DE LA CAPACITE A S'ORIENTER D'UNE SOURIS**

INSTRUMENTIERTE VORRICHTUNG ZUR CHARAKTERISIERUNG DER KAPAZITÄT EINER MAUS ZUR SELBSTORIENTIERUNG

INSTRUMENTED DEVICE FOR CHARACTERISING THE CAPACITY OF A MOUSE TO ORIENTATE ITSELF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.07.2015 FR 1557093**

(43) Date de publication de la demande:
**13.06.2018 Bulletin 2018/24**

(73) Titulaires:
• **Université de Montpellier**
  **34090 Montpellier (FR)**
• **INSERM - Institut National de la Santé et de la Recherche Médicale**
  **75013 Paris (FR)**
• **Ecole Pratique des Hautes Etudes**
  **75014 Paris (FR)**

(72) Inventeur: **MAURICE, Tangui**
**34980 Saint-Gely-Du-Fesc (FR)**

(74) Mandataire: **IPAZ**
**18 rue de la République**
**34000 Montpellier (FR)**

(56) Documents cités:
**CN-A- 101 884 306     CN-U- 201 995 441**
**US-A1- 2013 233 249**

**Description**

**Domaine technique**

La présente invention concerne un dispositif et procédé pour l'étude de la capacité à s'orienter d'au moins une souris dans un labyrinthe mettant en jeu les mémoires topographique et spatiale.

La présente invention se situe dans le domaine de l'instrumentation en laboratoire, et plus particulièrement celle destinée aux analyses comportementales et à l'apprentissage d'animaux de laboratoires.

**Etat de la technique antérieure**

De manière connue, un signe précurseur du développement d'un vieillissement pathologique ou de démence, par exemple de type Alzheimer, est la désorientation dans un environnement familier. Cependant, aucune procédure comportementale dans les études animales n'aborde actuellement la caractérisation de la capacité d'un tel individu à s'orienter dans un environnement familier, c'est-à-dire auquel l'animal a été au préalablement habitué pendant une durée conséquente.

On connait un dispositif comprenant une plateforme centrale et différentes boîtes contenant de la nourriture, reliées à la plateforme centrale par un conduit muni d'une porte opaque (CN 101 884 306A).

On connait aussi un dispositif d'entraînement de la mémoire des animaux constituant un labyrinthe et comprenant un dispositif rotatoire central, associé à un moteur (US 2013/0233249).

On connaît également un dispositif de type labyrinthe pour les rats, comprenant plusieurs rangées de cases d'entraînement empilées, les cages adjacentes communiquant entre elles, notamment par une échelle (CN 201995441 U).

On connait le document G. Winocur et al. Neurobiology of Aging, 31 (2010), 143-150, « A study of remote spatial memory in aged rats » dans lequel une population de rats est introduite dans un complexe environnemental comprenant une pluralité de recoins permettant de satisfaire un besoin particulier des rats (jouer, manger, boire, interagir). Ce document ne permet pas de tester les facultés d'orientation des rats à l'intérieur du complexe environnemental car toutes les cellules sont systématiquement visibles par les rats. Par ailleurs, les différentes activités proposées aux rats dans les cellules ne sont pas enregistrées et aucune donnée quantitative liée à l'utilisation desdites activités n'est mesurée. Enfin, ce test n'a été décrit que pour les rats et n'est pas adapté aux souris. Il a été utilisé jusqu'ici dans des études pharmacologiques après lésion hippocampique et non dans un contexte de maladie d'Alzheimer.

En effet, les tests comportementaux aujourd'hui utilisés (test d'activité, de mémoire, d'anxiété, de réponse au stress, d'addiction, etc...) dépendent étroitement de l'espèce animale utilisée, voire de la souche utilisée dans l'espèce considérée. Ainsi, les tests destinés aux rats et aux souris diffèrent très souvent, la souris étant par exemple plus mobile et exploratrice que le rat, ce dernier étant par ailleurs plus compétent dans les procédures. La mesure de réponses comportementales complexes demande donc l'élaboration de nouveaux tests comportementaux particulièrement adaptés à l'espèce considérée.

Différents tests de comportement animal existent aujourd'hui en neuropharmacologie comportementale. Mais aucun n'adresse spécifiquement la mémoire topographique chez la souris. Les tests de référence adressent la mémoire spatiale, comme l'apprentissage en piscine circulaire popularisé par Richard Morris, ou l'apprentissage en labyrinthe radial à huit bras qui permet une analyse simultanée des erreurs de mémoire de référence et de mémoire de travail. Ces tests ont l'avantage d'être utilisés chez le rat et la souris. Mais ils sont essentiellement pertinents pour mesurer des défauts d'apprentissage.

La présente invention a pour objet de répondre au moins en grande partie aux problèmes précédents et de conduire en outre à d'autres avantages.

Un autre but de l'invention est d'adresser directement un test utilisable sur l'ensemble des lignées de souris transgéniques modélisant la maladie d'Alzheimer. Un autre but de l'invention est de mesurer plus efficacement et plus précisément les facultés d'orientation d'une population de souris.

Un autre but de l'invention est d'adresser un test permettant de caractériser l'autisme, et/ou le comportement alimentaire, et/ou l'interaction sociale et/ou les sciences du mouvement et/ou l'état de dépression d'une population de souris.

Un autre but de l'invention est d'offrir une nouvelle plateforme automatisée d'analyse du comportement des souris dans un environnement complexe.

**Exposé de l'invention**

On atteint au moins l'un des objectifs précités avec un dispositif pour l'étude de la mémoire topographique d'au moins une souris, ledit dispositif comprenant :

- un espace central, dit agora, ouvert sur au moins un côté,
- une pluralité d'espaces clos, dits maisons, situées à la périphérie de l'agora, lesdites maisons étant ouvertes sur un côté et reliées à au moins un espace de circulation, dit rue, permettant à l'au moins une souris de rejoindre l'agora,
- une pluralité de moyens de stimulation de l'au moins une souris situés dans la pluralité de maisons,

lesdits au moins un espace de circulation formant un labyrinthe qui est agencé de telle sorte que les distances entre l'agora et chaque maison sont identiques, chaque maison étant accessible depuis l'agora par au moins deux chemins directs de même longueur, l'ensemble des chemins directs reliant chaque maison à l'agora étant de longueur égale. Eventuellement, ces espaces de circulations peuvent être fermés à certaines positions définies par des portes, afin d'analyser la stratégie d'exploration mise en jeu par les souris.

Il est ainsi possible de mettre une population de souris à l'intérieur d'une structure particulière qui stimulera leurs facultés d'orientation, leurs mémoires topographiques de ladite structure et plus particulièrement des maisons dans lesquelles les souris trouveront des activités et des récompenses. Préférentiellement, 6 à 8 individus sont placés dans ladite structure.

L'agora est un espace central non couvert et ouvert sur au moins un côté vers les espaces de circulation du dispositif. C'est dans l'agora que les souris sont placées en début d'expérience.

Les maisons sont situées tout autour de l'agora, et à une distance égale de celui-ci, de manière à ce que la longueur des chemins possibles pour s'y rendre soit identique pour toutes les maisons en partant du centre du dispositif. Ainsi, la probabilité qu'une souris saine et ne connaissant pas déjà le dispositif se rende dans l'une quelconque des maisons est identique.

Un chemin direct est constitué de la plus petite combinaison de rues empruntées permettant de se rendre d'un point à un autre. Un chemin direct est celui emprunté par l'au moins une souris pour parvenir le plus rapidement possible à une destination donnée, l'une des maisons, sans faire demi-tour et/ou sans emprunter une rue qui ne mène pas à l'endroit désiré.

Les espaces de circulations peuvent être fermés temporairement ou définitivement à certaines positions et à l'aide de portes, afin d'analyser la stratégie d'exploration mise en jeu par l'au moins une souris.

De préférence, toutes les maisons sont situées sur un seul niveau, et/ou réparties autour de l'agora suivant un plan en étoile, chaque maison étant située à une extrémité de ladite étoile. De manière avantageuse, l'agora est organisée suivant un plan en étoile à cinq branches.

Plus particulièrement, le dispositif selon le premier aspect de l'invention comprend par ailleurs :

- une pluralité de capteurs, préférentiellement situés dans les maisons, et agencés pour mesurer l'activité de l'au moins une souris avec lesdits moyens de stimulation, et

- une unité de traitement programmée pour déterminer des paramètres comportementaux de l'au moins une souris dans ledit dispositif à partir des mesures réalisées par la pluralité de capteurs.

L'unité de traitement est programmée pour traiter les informations renvoyées par les différents capteurs et/ou instruments et/ou caméras du dispositif. Préférentiellement, le traitement desdites informations renvoyées est réalisé en temps réel. L'unité de traitement est programmée pour enregistrer les informations collectées selon un protocole particulier permettant de sélectionner des informations d'intérêt parmi l'ensemble des données instrumentales. A titre d'exemples non limitatifs, le protocole permet à l'utilisateur de visualiser des aires d'intérêt sur le dispositif, des seuils de suivi des souris, des durées d'acquisition, des fréquences d'acquisition des capteurs et/ou les fréquences d'échantillonnage des caméras.

De manière avantageuse, le dispositif peut comprendre en outre une unité de contrôle agencée pour modifier les paramètres de fonctionnement des capteurs et/ou caméras et/ou sources lumineuses et/ou instruments dudit dispositif. L'unité de contrôle est reliée à au moins une partie des caméras et/ou sources lumineuses et/ou capteurs et/ou instruments dudit dispositif par des moyens filaires ou non filaires, et est configurée pour transmettre des paramètres de configuration et/ou recevoir des données.

L'activité des souris dans le dispositif est mesurée, à la fois dans les différentes maisons et dans les espaces de circulations à l'aide de la pluralité de capteurs situés dans le dispositif, permettant ainsi de collecter des données quantifiées ou quantifiables en relation avec le comportement des souris dans le dispositif. A titre d'exemples non limitatifs, les paramètres suivants permettent *in fine* de caractériser de manière quantifiable certains aspects du comportement des souris et de leur faculté d'orientation dans le dispositif :

- le nombre d'entrées de chaque souris dans chaque maison,

- le temps de trajet pour aller vers les différentes maisons,

- le nombre d'erreurs pour se rendre dans les différentes maisons, une erreur étant considérée comme le fait de s'engager dans une rue ne correspondant pas au chemin le plus court pour s'y rendre,

- le chemin total parcouru dans les espaces de circulation,

- le nombre d'entrées dans chacun des espaces de circulation,

- la localisation de chaque souris dans le dispositif,

- la locomotion des souris,

- les vitesses de déplacement de chaque souris,

- les interactions entre les différentes souris présentes dans le dispositif,

- le nombre et la position des demi-tours réalisés par chaque souris dans les espaces de circulation,

- le nombre et la position des redressements de chaque souris dans le dispositif,

- le nombre et la position des toilettages de chaque souris dans le dispositif,

- le temps passé dans chaque maison.

Il peut s'agir de paramètres directement mesurés par des capteurs, le suivi vidéo ou extraits des données mesurées et calculées ensuite.

Pour ce faire, les souris y sont introduites préférentiellement selon un protocole particulier comprenant deux phases :

- une première phase d'apprentissage au cours de laquelle les souris explorent librement le dispositif et les différentes maisons, et préférentiellement en groupe de plusieurs individus (par exemple 6-8). Cette première phase permet ainsi aux souris introduites dans le dispositif de mémoriser l'emplacement des différentes maisons, les différents chemins qui les y mènent et de connaître le type d'activité qu'elles y trouveront ;

- une deuxième phase de simulation de la mémoire topographique des souris au cours de laquelle elles sont mises dans un état tel qu'elles recherchent préférentiellement à rejoindre une maison particulière.

La première phase d'apprentissage comprend plusieurs sessions de durée variables. Par exemple, la phase d'apprentissage peut comprendre une, quatre, huit ou seize sessions de durées respectivement égales à un jour, une semaine, deux semaines ou quatre semaines.

La deuxième phase a lieu préférentiellement un certain temps après la dernière session d'apprentissage (par exemple 24 heures, 48 heures ou sept jours). Elle peut comprendre plusieurs sessions de tests, de durées variables et dans des conditions expérimentales différentes.

Les conditions expérimentales permettent de générer un état de manque particulier des souris, et notamment vis-à-vis de l'une des activités ou de l'un des stimuli qu'elles peuvent trouver dans le dispositif. Par exemple, les souris peuvent être privées de boisson ou de nourriture pendant quelques heures (par exemple de l'ordre de 20 h).

Les souris peuvent aussi être soumises à un traitement particulier afin de provoquer des déficiences particulières, de préférence en relation avec leur mémoire et/ou leur capacité à s'orienter. A titre d'exemple non limitatif, la qualité de la mémoire topographique des souris peut être testée par l'induction d'un état pathologique, dans les modèles non-transgéniques de la maladie d'Alzheimer ou à un âge auquel des animaux transgéniques développent les premiers symptômes, comme par exemple des animaux sur-exprimant des formes mutées de l'APP humaine (APP pour Amyloid Protein Precursor) et déclenchant spontanément la maladie d'Alzheimer.

Certaines souris peuvent aussi réaliser la deuxième phase sans avoir été soumises à un stress quelconque afin de mieux mettre en évidence certaines différences comportementales.

Durant la deuxième phase, les souris peuvent être réintroduites individuellement ou collectivement dans le dispositif.

Un dispositif selon l'invention est adapté pour l'étude de souris de tout type de lignées, telles que par exemple C57BL/6 ou Swiss.

Plus particulièrement, la pluralité de moyens de stimulation comprend au moins un moyen pour :

- donner à manger à l'au moins une souris, et/ou

- donner à boire à l'au moins une souris, et/ou

- faire jouer l'au moins une souris, et/ou

- faire courir l'au moins une souris, et/ou

- mettre l'au moins une souris en présence d'une autre souris,

- chaque moyen de stimulation étant mis en œuvre à l'intérieur d'une maison, et chaque maison ne comprenant qu'un seul type de moyen de stimulation.

Il est ainsi possible de stimuler l'au moins une souris selon des stimuli bien définis et indépendants, permettant ainsi de composer une pluralité de situations de stress et de mesurer l'influence d'une ou plusieurs privations sur le comportement général de l'au moins une souris, et plus particulièrement sur sa capacité à s'orienter dans le dispositif.

Il est ainsi possible à l'aide du présent dispositif de réaliser des tests permettant de caractériser l'autisme, et/ou le comportement alimentaire des souris, et/ou leur interaction sociale et/ou les sciences du mouvement et/ou l'état de dépression d'une population de souris.

De manière préférentielle, la pluralité de moyens de stimulation peut comprendre un distributeur de nourriture et la pluralité de capteurs peut comprendre au moins un capteur agencé pour mesurer le volume de nourriture mangé par l'au moins une souris et/ou le nombre d'utilisation dudit distributeur de nourriture et/ou le temps passé à l'utiliser.

Par ailleurs, le temps de trajet pour aller vers la maison comprenant le moyen pour donner à manger à l'au moins une souris ainsi que le nombre d'erreurs pour s'y rendre sont deux paramètres mesurés et calculés.

Dans un mode de réalisation particulier, le distributeur de nourriture peut comprendre :

- un réservoir principal à nourriture,

- un réservoir d'alimentation relié au réservoir principal, et

- une trappe (motorisée ou non) située entre le réservoir principal et le réservoir d'alimentation, ladite trappe étant agencée pour s'ouvrir et se fermer automatiquement en fonction du niveau de nourriture présent dans le réservoir d'alimentation.

De manière avantageuse et applicable aux différents modes de réalisation de l'invention, la pluralité de moyens de stimulation comprend au moins un distributeur de boisson et la pluralité de capteurs comprend au moins un capteur agencé pour mesurer le volume de liquide bu par l'au moins une souris et/ou le nombre d'utilisation de chaque distributeur de boisson et/ou le temps passé à les utiliser.

Par ailleurs, le temps de trajet pour aller vers la maison comprenant le moyen pour donner à boire à l'au moins une souris ainsi que le nombre d'erreurs pour s'y rendre sont deux paramètres mesurés et calculés.

Dans un mode particulier de réalisation, le moyen pour donner à boire à l'au moins une souris peut comprendre :

- au moins un réservoir contenant un liquide, et

- une tétine située à la base dudit réservoir.

Préférentiellement, le moyen pour donner à boire comprend au moins deux réservoirs afin de pouvoir réaliser une étude comparée de la prise d'eau plate ou d'eau sucrée par les souris et ainsi de mesurer leur état hédonique.

De manière préférentielle et applicable aux différents modes de réalisation de l'invention, la pluralité de moyens de stimulation comprend une plateforme de jeux comprenant au moins un tube agencé pour qu'au moins une souris puisse le traverser.

Avantageusement, l'au moins un tube est fait d'un matériau translucide afin de visualiser la souris qui s'y trouve à l'intérieur, comme par exemple du plexiglas, du verre ou un plastique transparent à la lumière infra-rouge.

Dans ce cas la pluralité de capteurs peut comprendre aussi une caméra pour mesurer l'activité de l'au moins une souris dans l'au moins un tube.

De manière avantageuse et applicable aux différents modes de réalisation de l'invention, la pluralité de moyens de stimulation comprend une plateforme de course comprenant au moins une roue d'activité.

Dans ce cas, la pluralité de capteurs peut comprendre aussi au moins un capteur pour mesurer le nombre de tours de roue réalisé et/ou la vitesse moyenne et/ou la vitesse instantanée et/ou le temps d'utilisation de chaque roue d'activité.

De manière préférentielle et compatible avec tous les modes de réalisation de l'invention, la pluralité de moyens de stimulation comprend une cage ajourée de dimensions aptes à accueillir une autre souris et agencée pour permettre à l'au moins une souris située dans la maison de toucher l'autre souris située dans ladite cage.

La cage est placée dans l'une des maisons d'une manière telle qu'une souris entrant dans la maison puisse interagir avec la souris enfermée dans la cage, et préférentiellement la toucher du bout du museau et/ou du bout d'au moins une patte.

La cage est préférentiellement faite d'un grillage dont la maille permet l'établissement de ce type de contacts physiques entre souris.

Dans ce cas, la pluralité de capteurs peut comprendre une caméra pour mesurer l'interaction de l'au moins une souris avec l'autre souris.

En particulier, les paramètres complémentaires suivants permettent *in fine* de caractériser de manière quantifiable certains aspects du comportement des souris et de leur faculté d'orientation dans le dispositif :

- le nombre et le volume de prises hydriques,

- le nombre et la quantité de prises alimentaires,

- la quantité de tours de roues, le temps d'activité physique,

- le temps d'interaction sociale,

- le temps passé dans les tunnels.

De manière particulière et applicable aux différents modes de réalisation de l'invention, la pluralité de capteurs comprend au moins un capteur pour détecter et mesurer les déplacements de l'au moins une souris à l'intérieur du labyrinthe. Pour ce faire, ledit au moins un capteur pour détecter et mesurer les déplacements de l'au moins une souris à l'intérieur du labyrinthe est agencé pour détecter et suivre l'ombre projetée de l'au moins une souris sur le sol du labyrinthe, par détermination de son centre de gravité par exemple, ou de son nez afin de suivre plus précisément ses interactions avec le dispositif d'une manière générale.

Ce capteur permet de mesurer plus particulièrement les déplacements de l'au moins une souris dans le labyrinthe et son attitude dans le labyrinthe (rues et/ou maisons). Comme exposé précédemment, ces mesures, et/ou celles réalisées au niveau de chaque maison et de chaque activité, permet de calculer un certain nombre de paramètres qui permettent, notamment par un traitement statistique des données mesurées pour chaque souris sur la pluralité de souris introduites dans le dispositif, de caractériser la qualité de la mémoire géographique de telles souris.

Par ailleurs, le temps de trajet pour aller vers les maisons comprenant respectivement le moyen pour donner à boire et à manger à l'au moins une souris ainsi que le nombre d'erreurs pour s'y rendre sont deux paramètres mesurés et calculés.

Dans ce cas, au moins une partie du sol dudit dispositif peut être faite d'un matériau transparent au rayonnement infrarouge.

Et préférentiellement le dispositif peut comprendre par ailleurs :

- au moins une source infrarouge située d'un premier côté du dispositif et agencée pour éclairer uniformément le sol dudit dispositif, et
- au moins une caméra sensible au rayonnement infrarouge située d'un second côté du dispositif et agencée pour mesurer les déplacements de l'au moins une souris dans le labyrinthe par détection de son ombre sur le sol dudit dispositif.

Il est ainsi possible de mesurer précisément les trajets de chaque souris à l'intérieur du dispositif, chaque souris pouvant être identifiées par ailleurs par un marqueur quelconque.

De manière préférentielle, les diodes émettant dans l'infrarouge sont agencées pour réaliser un rétroéclairage du sol du dispositif selon l'invention ; et la caméra infrarouge est positionnée à une hauteur suffisante pour suivre l'au moins une souris dans l'ensemble des éléments du dispositif. Le dispositif est préférentiellement constitué en matière plastique transparente au rayonnement infrarouge.

Selon un autre aspect de l'invention, applicable avec n'importe lequel des modes de réalisation du premier aspect, il est proposé un procédé d'évaluation de la capacité d'orientation d'au moins une souris dans le dispositif selon l'invention, ledit procédé comprenant :

EP 3 331 353 B1

- au moins une étape d'apprentissage consistant en la mise en situation de ladite au moins une souris dans le dispositif pendant une durée prédéterminée dite d'apprentissage,
- une étape de privation hydrique et/ou de nourriture de ladite au moins une souris pendant une période dite de privation,
- une étape de test durant laquelle l'au moins une souris est réintroduite dans le dispositif,

l'unité de traitement détermine le nombre d'erreurs faites sur le chemin vers chaque maison et/ou le temps de parcours pour entrer dans chaque maison et/ou la trajectoire empruntée..

**Description des figures et des modes de réalisation**

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :

- la figure 1 illustre une vue du dessus du dispositif selon l'invention,
- la figure 2 illustre une vue de profil du dispositif selon l'invention,
- la figure 3 illustre les performances des animaux privés d'eau et testés 24 heures après leur habituation au dispositif,
- la figure 4 illustre les performances des animaux privés de nourriture et testés 24 heures après leur habituation au dispositif,
- la figure 5 illustre les performances d'animaux sains privés d'eau et testés 24 heures après leur habituation au dispositif, puis contaminés par un modèle aigu de la maladie d'Alzheimer et re-testés après 7 jours ; ainsi qu'un indice de discrimination.

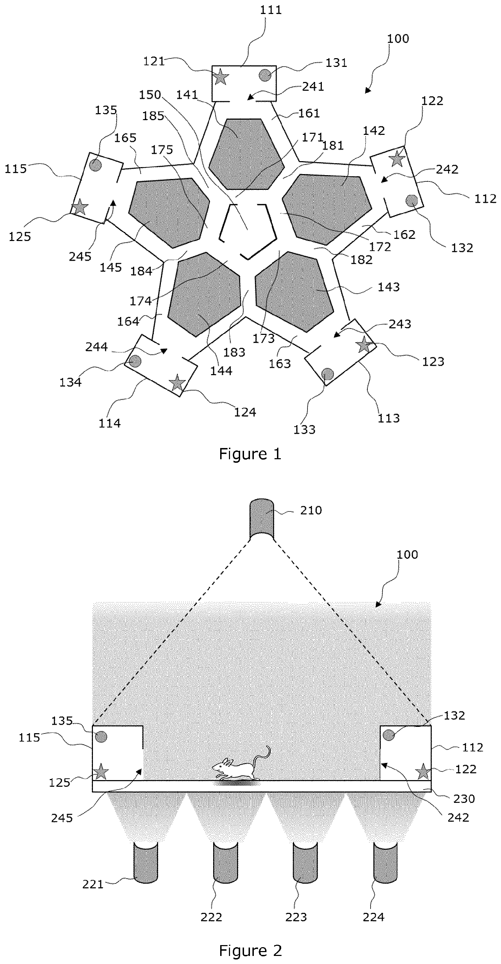

Les modes de réalisation qui seront décrits dans la suite ne sont nullement limitatifs.
En particulier toutes les variantes et tous les modes de réalisation décrits sont combinables entre eux si rien ne s'oppose à cette combinaison sur le plan technique.
Sur les figures, les éléments communs à plusieurs figures conservent la même référence.
En référence aux FIGURES 1 et 2, le dispositif 100 selon l'invention comprend une pluralité de maisons 111-115 - ici au nombre de cinq - réparties autour d'un espace central dénommé agora 150 et reliées entre elles par des moyens de circulation, dits rues, 161-165, 171-175, 181-185 formant un labyrinthe.
Le labyrinthe ainsi formé a préférentiellement une forme d'étoile à cinq branches dans laquelle les maisons 111-115 occupent chacune un sommet. Les obstacles 141-145 sont situés au milieu des branches de l'étoile et permettent de définir notamment deux boucles de circulation :

- une boucle de circulation extérieure comprenant les moyens de circulation 161-165 et définissant ainsi le plus court moyen de relier deux maisons successives ;

- une boucle de circulation intérieure 171-175 qui entoure l'agora 175 située au centre de l'étoile.

La boucle de circulation extérieure 161-165 est reliée à la boucle de circulation intérieure 171-175 par l'intermédiaire de coursives 181-185 qui permettent de contourner les obstacles 141-145.
Préférentiellement, les murs latéraux extérieurs des moyens de circulation 161-165 formant la boucle de circulation extérieure sont d'une hauteur telle que l'au moins une souris ne peut pas les franchir et sortir du dispositif 100.
Avantageusement, les murs latéraux extérieurs des moyens de circulation 161-165 formant la boucle de circulation extérieure sont réalisés dans un matériau translucide afin de pouvoir observer l'au moins une souris évoluer à l'intérieur et de permettre à l'animal de mettre en jeu sa mémoire spatiale sur ces circulations extérieures en se servant de repères extérieurs liés à la configuration de la pièce et du dispositif et renforcer ainsi sa mémorisation cartographique et/ou topographique dudit dispositif.
Ainsi les maisons 111-115 sont toutes situées à une égale distance de l'agora 150 et dispose du même nombre de chemins possibles pour les atteindre depuis l'agora 150, les chemins directs étant tous de même longueur. En d'autres termes, les maisons 111-115 sont disposées d'une manière telle que la probabilité de s'y rendre depuis l'agora 150 est la même pour chacune d'entre elles.
Les maisons 111-115 sont de tailles suffisantes pour qu'au moins une souris puisse s'y introduire et évoluer à l'intérieur. En particulier, elles sont d'une hauteur suffisante pour que l'au moins une souris puisse s'y tenir debout.
Avantageusement les dimensions latérales des maisons sont d'environ 280 mm x 160 mm, pour une hauteur de 300 mm.
Chaque maison 111-115 dispose d'une ouverture 241-245 permettant à l'au moins une souris de s'y introduire et d'en ressortir.

Chaque ouverture 241-245 de chaque maison 111-115 débouche sur un espace de circulation 161-165 permettant à l'au moins une souris de déambuler dans le dispositif 100.

Le dispositif 100 selon l'invention prend la forme d'un labyrinthe comprenant la pluralité d'espaces de circulation 161-165 et 171-175, les maisons 111-115 et des obstacles 141-145.

Dans le mode de réalisation illustré aux FIGURES 1 et 2, les obstacles 141-145 sont d'une hauteur telle que les souris, mêmes debout sur leurs pattes arrières, ne peuvent pas voir les maisons 111-115 de l'autre côté. Typiquement, la hauteur des obstacles est au moins égale à la hauteur des maisons, par exemple 300 mm.

Dans l'exemple illustré sur les FIGURES 1 et 2, chaque maison 111-115 comprend un moyen de stimulation pour souris. Ce moyen permet de stimuler au moins un sens de la souris et/ ou de lui permettre de réaliser une activité.

Ainsi, la maison 111 comprend au moins une roue d'activité 121 dans laquelle l'au moins une souris peut monter et courir, la roue tournant autour d'un axe de rotation fixe. La maison 111 est dénommée RUN dans les paragraphes suivants.

Additionnellement, la maison 111 peut comprendre aussi au moins un capteur 131 pour mesurer l'activité de la souris avec ladite roue d'activité. Typiquement, mais non limitativement, l'au moins un capteur 131 peut être du type capteur de vitesse monté sur la roue d'activité afin de mesurer la vitesse de rotation moyenne et instantanée, l'accélération, un compte tour pour mesurer le nombre de tours réalisés et un chronomètre pour mesurer le temps d'utilisation de ladite roue d'activité. Un système basé sur la Spontaneous activity Wheel de Bioseb peut être envisagé (www.bioseb.com/bioseb/anglais/default/item id=40.php).

La maison 112 comprend une cage 122 occupant au moins une partie de l'espace de ladite maison 112, et dans laquelle une souris est introduite. Préférentiellement, il s'agit d'une souris d'un autre sexe que celles évoluant dans le dispositif 100. La cage 122 est agencée pour que la souris qui y est enfermée puisse interagir avec l'au moins une souris qui entre dans la maison 122. Typiquement, la cage 122 comprend un grillage dont les mailles sont suffisamment espacées pour que les souris puisse y faire passe l'extrémité de leur museau et/ou établir un contact physique entre elles, de part et d'autre de ladite cage 122. La maison 112 est dénommée INTERACT dans les paragraphes suivants.

Additionnellement, la maison 112 peut comprendre aussi au moins un capteur 132 pour mesurer l'activité de la souris dans ladite maison 112, et plus particulièrement son interaction avec la souris enfermée dans la cage 112. Typiquement, mais non limitativement, l'au moins un capteur 132 peut être du type caméra afin de pouvoir étudier le comportement des souris dans la maison 112, et/ou d'identifier certaines attitudes particulières et révélatrices de certaines pathologies ou déviances comportementales, par exemple le temps et le nombre de contact directs entre une souris et l'animal placé dans le compartiment derrière la grille.

La maison 113 comprend au moins un tube d'activité 123 dans lequel l'au moins une souris peut monter et marcher et qu'elle peut traverser librement. De manière préférentielle, l'au moins un tube est situé à l'horizontale et/ou légèrement surélevé par rapport au sol. La maison 113 est dénommée PLAY dans les paragraphes suivants.

Additionnellement, la maison 113 peut comprendre aussi au moins un capteur 133 pour mesurer l'activité de la souris avec l'au moins un tube d'activité 123, par exemple en plexiglas transparent. Typiquement, mais non limitativement, l'au moins un capteur 133 peut être du type caméra afin d'étudier le comportement de l'au moins une souris dans le tube et/ou certaines attitudes particulières et révélatrices de certaines pathologies ou déviances comportementales, par exemple le temps passé dans l'au moins un tube et/ou le nombre de fois que l'au moins une souris entre dans ledit tube.

La maison 114 comprend un dispenseur de nourriture 124 agencé pour qu'une souris puisse y manger en toute autonomie. Préférentiellement, le dispenseur de nourriture 124 est du type distributeur automatique de granulés, comprenant un réservoir principal à granulés comestibles, un réservoir secondaire connecté au réservoir principal par l'intermédiaire d'une porte automatique qui permet son remplissage lorsque le réservoir secondaire est vide ou en dessous d'un certain seuil. L'au moins une souris peut ainsi manger librement dans le réservoir secondaire. La maison 114 est dénommée EAT dans les paragraphes suivants.

Additionnellement, la maison 114 peut comprendre aussi au moins un capteur 134 pour mesurer l'activité de la souris avec le dispenseur 124. Typiquement, mais non limitativement, l'au moins un capteur 134 peut être agencé pour mesurer le volume de nourriture ingurgité par l'au moins une souris à chaque prise, le nombre de fois qu'elle utilise ledit dispenseur 124 et/ou son temps d'utilisation. Par ailleurs, l'au moins un capteur 134 permet aussi de mesurer chaque prise alimentaire (chaque bouchée), son horodatage, sa durée, la quantité ingérée. Un système automatisé type BioDAQ de chez Research Diets Inc. peut être utilisé (http://www.researchdiets.com/biodaq/applications/food-water-intake).

Enfin, la maison 115 comprend au moins un dispenseur de liquide 125. Préférentiellement, la maison 115 comprend deux dispenseurs 125 afin de pouvoir réaliser des mesures d'hédonie, le premier dispenseur comprenant par exemple une solution d'eau sucrée et le deuxième dispenseur comprenant une solution d'eau plate. Préférentiellement, le dispenseur 125 contient un liquide comestible par l'au moins une souris. Le dispenseur 125 peut comprendre un réservoir agencé pour contenir un volume donné de liquide et une pipette pour permettre à l'au moins une souris de boire de manière autonome. La maison 115 est dénommée DRINK dans les paragraphes suivants.

Additionnellement, la maison 115 peut comprendre aussi au moins un capteur 125 pour mesurer l'activité de la souris avec le dispenseur 125 de liquide. Typiquement, mais non limitativement, l'au moins un capteur 125 peut être agencé pour mesurer le volume de chaque prise hydrique de l'au moins une souris, le nombre de fois qu'elle utilise ledit dispenseur 125 et/ou sont temps d'utilisation. Un système automatisé type BioDAQ de chez Research Diets Inc. peut être utilisé (http://www.researchdiets.com/biodaq/applications/food-water-intake).

Comme illustré sur la FIGURE 2, le dispositif 100 selon l'invention peut comprendre aussi au moins un capteur dit d'environnement 210 agencé pour mesurer l'activité générale de l'au moins une souris dans le dispositif 100, et notamment ses déplacements dans le labyrinthe.

De manière avantageuse, l'au moins un capteur 210 peut être de type caméra, dont le champ couvre au moins en partie le dispositif 100.

De manière astucieuse, le déplacement de l'au moins une souris peut être mesuré par une technique basée sur le repérage de l'ombre portée de l'au moins une souris sur le sol 230. Pour ce faire, au moins une source d'éclairage 221-224 est disposée d'un côté du dispositif 100 de manière à éclairer le sol de ce dernier de manière homogène.

Préférentiellement, l'au moins une source d'éclairage 221-224 est située sous le sol dudit dispositif.

Avantageusement, l'au moins une source d'éclairage 221-224 est du type source infrarouge afin que les souris ne soient pas perturbées et/ou influencées par l'éclairement du sol 230.

Dans ce mode de réalisation particulier, le sol 230 est transparent au rayonnement émis par l'au moins une source d'éclairage 221-224, et l'au moins un capteur d'environnement 210 est sensible au rayonnement infrarouge. Ainsi, lorsque l'au moins une souris se déplace sur le sol, le capteur 210 perçoit moins de lumière à la position où se trouve ladite souris. Il est donc possible de mesurer efficacement et à moindre coût le déplacement de l'au moins une souris dans tout le labyrinthe.

Le dispositif comprend aussi une unité de traitement qui permet d'exploiter l'ensemble des données mesurées par les différents capteurs 121-125 situés dans chaque maison 111-115, ainsi que par l'au moins un capteur d'environnement 210. Les données mesurées sont collectées selon un protocole défini par l'utilisateur (dessins des aires d'intérêt, des seuils de suivi des animaux, de la durée de l'acquisition, des intervalles de temps de recueil d'information, etc...). Les données finales sont préférentiellement présentées sous la forme d'un tableur sous un format très versatile pour permettre à l'utilisateur d'appliquer ses propres macros de calcul.

A titre d'exemples non limitatifs, les paramètres suivants peuvent être calculés à partir des données de l'ensemble des capteurs 121-125, 210 :

- Le nombre d'entrées dans chacune des maisons 111-115 et dans l'agora 150.

- Le temps mis à entrer dans les maisons 111-115, et plus particulièrement dans celles où l'au moins une souris peut boire 115 et manger 114.

- Le nombre d'erreurs pour se rendre dans les maisons 111-115, et plus particulièrement dans celles où l'au moins une souris peut boire 115 et manger 114.

- L'ensemble des trajets empruntés par chaque souris présente dans le dispositif 100.

- Nombres d'entrées dans chacun des espaces de circulation 161-165, 171-175 et 181-185.

- La vitesse de progression de chaque souris dans le dispositif 100.

- L'interaction entre chaque souris présentes dans le dispositif 100.

- Le nombre et le lieu des redressements de chaque souris.

- Le nombre et le lieu des toilettages de chaque souris.

- Le nombre et la position des changements de trajectoires de chaque souris dans le dispositif 100 (demi-tours).

- le nombre et le volume de prises hydriques, et le temps passé dans la maison correspondante,

- le nombre et la quantité de prises alimentaires, et le temps passé dans la maison correspondante,

- la quantité de tours de roues, le temps d'activité physique, et le temps passé dans la maison correspondante,

- le temps d'interaction sociale et le temps passé dans la maison correspondante,

- le temps passé dans les tunnels et le temps passé dans la maison correspondante.

La présente invention concerne ainsi un dispositif pour l'étude de la capacité à s'orienter d'au moins une souris dans un labyrinthe, ledit dispositif comprenant une pluralité de maisons réparties autour d'une agora central et dans lesquelles des moyens de stimulation permettent à l'au moins une souris de réaliser certaines activité et/ou de satisfaire certains besoins primaires. Chaque maison comprend par ailleurs au moins un capteur pour mesurer l'activité de la souris dans ladite maison, et plus particulièrement son utilisation du moyen de stimulation. Enfin, un capteur permet de détecter et mesurer les déplacements de chaque souris dans le dispositif. Des paramètres quantitatifs issus de ces mesures permettent ainsi de mettre en avant la qualité de la mémoire géographique de l'au moins une souris.

Plusieurs exemples de tests et d'utilisation du dispositif selon l'invention vont maintenant être décrits :

• comportement des souris après 4 jours d'habituation : au cours d'une première série d'expérience, les souris ont été entrainées pendant 4 jours (4h par jour) en leur permettant de se déplacer librement dans le dispositif. 24h après le dernier entraînement, les animaux ont été placés individuellement dans le dispositif et leur exploration a été mesurée et analysée pendant 10 min. La latence mise pour entrer dans chaque maison, le nombre d'entrée et le temps total de présence dans chaque maison est reproduit dans le tableau n°1.

| Paramètre | Maisons | | | | | |
|---|---|---|---|---|---|---|
| | *Drink* | *Eat* | *Run* | *Interact* | *Play* | *Agora* |
| Latence | (s)93 $\pm$ 17 | 86 $\pm$ 26 | 49 $\pm$ 13 | 74 $\pm$ 25 | 120 $\pm$ 35 | |
| Durée (s) | 20 $\pm$ 4 | 67 $\pm$ 10 | 200 $\pm$ 38 | 88 $\pm$ 19 | 65 $\pm$ 16 | 11 $\pm$ 2 |
| Nb d'entrées | 2.8 $\pm$ 0.3 | 4.9 $\pm$ 0.9 | 7.3 $\pm$ 0.9 | 5.6 $\pm$ 0.8 | 4.3 $\pm$ 0.8 | 7.5 $\pm$ 1.1 |

Les souris tendent à explorer différentiellement les différentes maisons, avec le plus grand nombre d'entrées dans la maison Courir et les plus bas scores dans la maison Boire. Mais toutes les maisons sont bien reconnues par les animaux, suggérant qu'ils explorent efficacement toute la complexité de l'appareil.

• Mémorisation des souris dans le dispositif après 4 jours d'habituation : l'apprentissage topographique des souris est ensuite testé, ces dernières étant privées d'eau ou de nourriture pendant 20 h avant d'être introduites à nouveau dans le dispositif. Les périodes d'habituation ont varié de zéro à 1 jour, 1, 2 ou 4 semaines. Dans cette expérience, les performances des animaux ont été mesurées et analysées, et plus particulièrement le temps de latence pour entrer dans la maison DRINK ou EAT ainsi que le nombre d'erreurs faites entre l'agora et chacune des maisons.

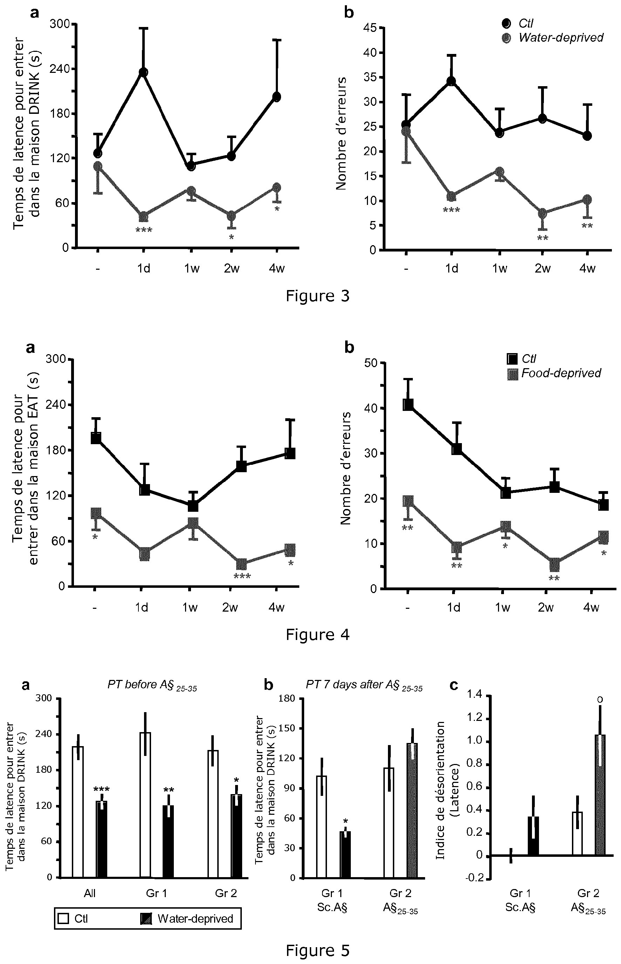

Les résultats pour les animaux privés de boisson sont présentés en FIGURE 3, ceux obtenus pour les animaux privés de nourriture sont présentés en FIGURE 4, en comparaison respectivement avec des animaux témoins (Ctl).

Comme visible sur la FIGURE 3, il apparaît que les animaux non privés montrent des performances moyennes, quelle que soit la période d'habituation, avec une latence moyenne de 156 s pour entrer dans la maison DRINK et un nombre moyen d'erreurs de 27 ($R^2$ = 0.012 pour la latence et $R^2$ = 0.183 pour le nombre d'erreurs). En revanche, les animaux privés d'eau montrent des latences et des nombres d'erreurs qui décroissent significativement avec la période d'habituation, et de manière significative après 1 jour, 2 ou 4 semaines d'habituation. Le profil des erreurs décroit significativement avec les sessions ($R^2$ = 0.563).

La FIGURE 4 illustre les performances pour les animaux privés de nourriture. Les animaux non-déprivés, montrent des performances moyennes quelle que soit la période d'habituation, en terme de latence pour entrer dans la maison EAT (moyenne de 148 s, $R^2$ = 0.002). Cependant et de façon surprenante, le nombre d'erreur décroît avec les sessions ($R^2$ = 0.847). En revanche, les animaux privés de nourriture montrent latences et des nombres d'erreurs significativement réduits, particulièrement après 2 ou 4 semaines d'habituation. Par ailleurs, latences et les nombres d'erreur sont inférieurs à celles et ceux des animaux de contrôles non-habitués. Les profils des mesures relatives aux animaux déprivés décroissent significativement avec les nombres de sessions ($R^2$ = 0.381 pour les latences et $R^2$ = 0.343 pour les nombres d'erreurs).

• Désorientation topographique suite à la toxicité amyloïde : l'objectif est ici de déterminer si un état pathologique de type Alzheimer peut induire chez les souris un oubli de la mémoire topographique. Pour ce faire, un lot d'animaux a été habitué pendant 2 semaines au dispositif. Puis leur apprentissage a été testé une première fois après privation d'eau. Enfin, une préparation d'oligomères de peptide Aβ25-35 leur a été injectée par voie intracérébroventriculaire. Cette préparation représente un modèle aigu de maladie d'Alzheimer très utilisé en laboratoire.

Les résultats sont illustrés sur la FIGURE 5. Pour les animaux ayant intégré l'emplacement de la maison DRINK et montrant le jour du test une latence significativement inférieure au groupe contrôle, non-déprivé, l'injection de peptide amyloïde induit une amnésie qui ne se voit pas pour le groupe traité avec le peptide contrôle. Ces derniers sont toujours aussi efficaces, voire plus, à se rappeler l'emplacement de la maison DRINK même 8 jours après la fin de l'habituation. Un calcul simple permet de définir un indice de désorientation (DI) :

$$DI = (PT7/PT0) - (PT7/PT0)_{groupe\ contrôle}$$

Avec PT7 = valeur du test de mémoire 7 jours après l'injection de peptide $A\beta_{25\text{-}35}$, PT0 = valeur du test de mémoire immédiatement avant l'injection d'$A\beta_{25\text{-}35}$.

Ainsi cet indice est nul pour les animaux contrôles (souris non privées d'eau et injectées avec un peptide Sc.AB non toxique) et significativement élevé chez les animaux traités avec le peptide amyloïde $A\beta_{25\text{-}35}$, permettant de proposer des études pharmacologiques pour tester si des molécules candidats médicaments atténuent ou bloquent l'augmentation de cet indice et, donc, la désorientation topographique.

L'habituation dans le dispositif selon l'invention constitue donc un environnement riche et complexe, qui affecte la plasticité cérébrale et la neurogenèse de l'hippocampe.

La présente invention permet ainsi de mesurer quantitativement le comportement animal, notamment dans le contexte de la neuropharmacologie et de la recherche animale préclinique, visant notamment à mesurer les processus d'apprentissage basé sur l'exploration latente spontanée d'un l'environnement complexe et des processus d'habituation / de familiarisation des souris avec cet environnement.

Le présent dispositif permet de mesurer des différences significatives entre le comportement des animaux habitués et celui des animaux non-habitués dans un test d'essai amnésique (pour l'eau ou la nourriture). La période d'habituation peut varier de un jour à 4 semaines ou sur une période plus longue.

Le présent dispositif est apte à mesurer l'impact de déficits cognitifs liés notamment à l'âge, à des modèles pharmacologiques d'amnésie (traitement de la scopolamine, par exemple), à des modèles pharmacologiques et génétiques de démence (comme illustré ici avec l'injection de peptide d'amyloïde, mais également des lignées transgéniques de la maladie d'Alzheimer) et d'autres conditions débilitantes (animaux stressés ou dépressifs, par exemple).

La présente invention permet ainsi notamment d'analyser l'un des signes d'alerte précurseur important pour la maladie d'Alzheimer : le symptôme de désorientation spatial.

Selon l'un des modes de réalisation, le dispositif permet de caractériser l'autisme.

Selon l'un des autres modes de réalisation, le dispositif permet de caractériser le comportement alimentaire.

Selon encore l'un des autres modes de réalisation, le dispositif permet de caractériser l'interaction sociale.

Selon encore l'un des autres modes de réalisation, le dispositif permet de caractériser les sciences du mouvement.

Selon encore l'un des autres modes de réalisation, le dispositif permet de caractériser l'état de dépression d'une population de souris.

Bien sûr, l'invention n'est pas limitée aux exemples qui viennent d'être décrits et de nombreux aménagements peuvent être apportés à ces exemples sans sortir du cadre de l'invention. Notamment, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Dispositif (100) pour l'étude de la mémoire topographique d'au moins une souris, ledit dispositif (100) comprenant :

   - un espace central, dit agora (150), ouvert sur au moins un côté,
   - une pluralité d'espaces clos, dits maisons (111-115), situées à la périphérie de l'agora (150), lesdites maisons (111-115) étant ouvertes sur un côté et reliées à au moins un espace de circulation, dit rue, (161-165, 171-175, 181-185) permettant à l'au moins une souris de rejoindre l'agora (150), et
   - une pluralité de moyens de stimulation (121-125) de l'au moins une souris situés dans la pluralité de maisons (111-115),

   caractérisé en ce que lesdits au moins un espace de circulation (161-165, 171-175, 181-185) forment un labyrinthe qui est agencé de telle sorte que les distances entre l'agora (150) et chaque maison (111-115) sont identiques, chaque maison (111-115) étant accessible depuis l'agora (150) par au moins deux chemins directs de même longueur, l'ensemble des chemins directs reliant chaque maison (111-115) à l'agora (150) étant de longueur égale.

2. Dispositif (100) selon la revendication précédente, caractérisé en ce qu'il comprend par ailleurs :

- une pluralité de capteurs (131-135) agencés pour mesurer l'activité de l'au moins une souris avec la pluralité de moyens de stimulation (121-125), et
- une unité de traitement agencée pour déterminer des paramètres comportementaux de l'au moins une souris dans ledit dispositif (100) à partir des mesures réalisées par la pluralité de capteurs (131-135).

3. Dispositif (100) selon la revendication précédente, **caractérisé en ce que** la pluralité de moyens de stimulation (121-125) comprend un distributeur de nourriture ; et **en ce que** la pluralité de capteurs (131-135) comprend au moins un capteur agencé pour mesurer le volume de nourriture mangé par l'au moins une souris et/ou le nombre d'utilisation dudit distributeur de nourriture et/ou le temps passé à l'utiliser.

4. Dispositif (100) selon la revendication précédente, **caractérisé en ce que** le distributeur de nourriture comprend :

   - un réservoir principal à nourriture,
   - un réservoir d'alimentation relié au réservoir principal, et
   - une trappe située entre le réservoir principal et le réservoir d'alimentation, ladite trappe étant agencée pour s'ouvrir et se fermer automatiquement en fonction du niveau de nourriture présent dans le réservoir d'alimentation.

5. Dispositif (100) selon l'une quelconque des revendications 2 à 4, **caractérisé en ce que** la pluralité de moyens de stimulation (121-125) comprend au moins un distributeur de boisson ; et **en ce que** la pluralité de capteurs (131-135) comprend au moins un capteur agencé pour mesurer le volume de liquide bu par l'au moins une souris et/ou le nombre d'utilisation de chaque distributeur de boisson et/ou le temps passé à les utiliser.

6. Dispositif (100) selon la revendication précédente, **caractérisé en ce que** le distributeur de boisson comprend :

   - au moins un réservoir contenant un liquide, et
   - une tétine située à la base dudit réservoir.

7. Dispositif (100) selon l'une quelconque des revendications 2 à 6, **caractérisé en ce que** la pluralité de moyens de stimulation (121-125) comprend une plateforme de jeux comprenant au moins un tube agencé pour qu'au moins une souris puisse le traverser ; et **en ce que** la pluralité de capteurs (131-135) comprend une caméra pour mesurer l'activité de l'au moins une souris dans l'au moins un tube.

8. Dispositif (100) selon l'une quelconque des revendications 2 à 7, **caractérisé en ce que** la pluralité de moyens de stimulation (121-125) comprend une plateforme de course comprenant au moins une roue d'activité ; et **en ce que** la pluralité de capteurs (131-135) comprend au moins un capteur pour mesurer le nombre de tours de roue réalisé et/ou la vitesse moyenne et/ou la vitesse instantanée et/ou le temps d'utilisation de chaque roue d'activité.

9. Dispositif (100) selon l'une quelconque des revendications 2 à 8, **caractérisé en ce que** la pluralité de moyens de stimulation (121-125) comprend une cage ajourée de dimensions aptes à accueillir une autre souris et agencée pour permettre à l'au moins une souris située dans la maison (111-115) de toucher l'autre souris située dans ladite cage ; et **en ce que** la pluralité de capteurs (131-135) comprend une caméra pour mesurer leur interaction.

10. Dispositif (100) selon l'une quelconque des revendications 2 à 9, **caractérisé en ce que** la pluralité de capteurs (131-135) comprend au moins un capteur (210) pour détecter et mesurer les déplacements de l'au moins une souris à l'intérieur du labyrinthe.

11. Dispositif (100) selon la revendication précédente, **caractérisé en ce qu'**au moins une partie du sol (230) dudit dispositif (100) est faite d'un matériau transparent au rayonnement infrarouge.

12. Dispositif (100) selon la revendication précédente, **caractérisé en ce qu'**il comprend par ailleurs :

   - au moins une source infrarouge (221-224) située d'un premier côté du dispositif (100) et agencée pour éclairer uniformément le sol (230) dudit dispositif (100),
   - au moins une caméra (210) sensible au rayonnement infrarouge située d'un second côté du dispositif (100) et agencée pour mesurer les déplacements de l'au moins une souris dans le labyrinthe par détection de son ombre sur le sol dudit dispositif (100).

13. Procédé d'évaluation de la capacité d'orientation d'au moins une souris dans le dispositif (100) selon l'une quelconque des revendications 10 à 12, ledit procédé comprenant :

- au moins une étape d'apprentissage consistant en la mise en situation de ladite au moins une souris dans le dispositif (100) pendant une durée prédéterminée dite d'apprentissage,
- une étape de privation hydrique et/ou de nourriture de ladite au moins une souris pendant une période dite de privation,
- une étape de test durant laquelle l'au moins une souris est réintroduite dans le dispositif (100),

l'unité de traitement détermine le nombre d'erreurs faites sur le chemin vers chaque maison (111-115), et/ou le temps de parcours pour entrer dans chaque maison (111-115), et/ou la trajectoire empruntée.


**Patentansprüche**

1. Vorrichtung (100) zum Untersuchen des topografischen Gedächtnisses wenigstens einer Maus, wobei die Vorrichtung (100) umfasst:

   - einen zentralen Raum, sogenannte Agora (150), der an wenigstens einer Seite offen ist,
   - eine Vielzahl von geschlossenen Räumen, sogenannten Häusern (111-115), die sich am Umkreis der Agora (150) befinden, wobei die Häuser (111-115) auf einer Seite offen sind und mit wenigstens einem Verkehrsraum, sogenannten Straße (161-165, 171-175, 181-185) verbunden sind, welcher der wenigstens einen Maus ermöglicht, zu der Agora (150) zu gelangen, und
   - eine Vielzahl von Mitteln zur Stimulation (121-125) der wenigstens einen Maus, die sich in der Vielzahl von Häusern (111-115) befinden,

   **dadurch gekennzeichnet, dass** der wenigstens eine Verkehrsraum (161-165, 171-175, 181-185) ein Labyrinth bildet, das derart eingerichtet ist, dass die Abstände zwischen der Agora (150) und jedem Haus (111-115) identisch sind, wobei jedes Haus (111-115) von der Agora (150) aus über wenigstens zwei direkte Wege gleicher Länge zugänglich ist, wobei alle direkten Wege, die jedes Haus (111-115) mit der Agora (150) verbinden, von gleicher Länge sind.

2. Vorrichtung (100) nach dem vorhergehenden Anspruch, **dadurch gekennzeichnet, dass** sie außerdem umfasst

   - eine Vielzahl von Sensoren (131-135), die dazu eingerichtet sind, die Aktivität der wenigstens einen Maus mit der Vielzahl von Stimulationsmitteln (121-125) zu messen, und
   - eine Verarbeitungseinheit, die dazu eingerichtet ist, Verhaltensparameter der wenigstens einen Maus in der Vorrichtung (100) anhand der Messungen, die durch die Vielzahl von Sensoren (131-135) durchgeführt werden, zu bestimmen.

3. Vorrichtung (100) nach dem vorhergehenden Anspruch, **dadurch gekennzeichnet, dass** die Vielzahl von Stimulationsmitteln (121-125) einen Nahrungsspender umfassen; und dass die Vielzahl von Sensoren (131-135) wenigstens einen Sensor umfasst, der dazu eingerichtet ist, das von der wenigstens einen Maus verzehrte Nahrungsvolumen und/oder die Anzahl der Benutzung des Nahrungsspenders und/oder die für dessen Benutzung verstrichene Zeit zu messen.

4. Vorrichtung (100) nach dem vorhergehenden Anspruch, **dadurch gekennzeichnet, dass** der Nahrungsspender umfasst:

   - einen Hauptnahrungsbehälter,
   - einen mit dem Hauptbehälter verbundenen Versorgungsbehälter und
   - eine Klappe, die sich zwischen dem Hauptbehälter und dem Versorgungsbehälter befindet, wobei die Klappe dazu eingerichtet ist, sich in Abhängigkeit von dem Nahrungsfüllstand in dem Versorgungsbehälter automatisch zu öffnen und zu schließen.

5. Vorrichtung (100) nach einem der Ansprüche 2 bis 4, **dadurch gekennzeichnet, dass** die Vielzahl von Stimulationsmitteln (121-125) wenigstens einen Getränkespender umfasst; und dass die Vielzahl von Sensoren (131-135) wenigstens einen Sensor umfasst, der dazu eingerichtet ist, das von der wenigstens einen Maus getrunkene Flüssigkeitsvolumen und/oder die Anzahl der Benutzung eines jeden Getränkespenders und/oder die für deren Benutzung verstrichene Zeit zu messen.

6. Vorrichtung (100) nach dem vorhergehenden Anspruch, **dadurch gekennzeichnet, dass** der Getränkespender umfasst:

  - wenigstens einen Behälter, der eine Flüssigkeit enthält, und
  - einen Sauger, der sich an der Basis des Behälters befindet,

7. Vorrichtung (100) nach einem der Ansprüche 2 bis 6, **dadurch gekennzeichnet, dass** die Vielzahl von Stimulationsmitteln (121-125) eine Spielplattform umfasst, die wenigstens ein Rohr aufweist, das dazu eingerichtet ist, dass wenigstens eine Maus es durchqueren kann; und dass die Vielzahl von Sensoren (131-135) eine Kamera umfasst, um die Aktivität der wenigstens einen Maus in dem wenigstens einen Rohr zu messen.

8. Vorrichtung (100) nach einem der Ansprüche 2 bis 7, **dadurch gekennzeichnet, dass** die Vielzahl von Stimulationsmitteln (121-125) eine Laufplattform umfasst, die wenigstens ein Aktivitätsrad umfasst; und dass die Vielzahl von Sensoren (131-135) wenigstens einen Sensor umfasst, um die vollzogene Anzahl von Radumdrehungen und/oder die Durchschnittsgeschwindigkeit und/oder die momentane Geschwindigkeit und/oder die Benutzungszeit eines jeden Aktivitätsrades zu messen.

9. Vorrichtung (100) nach einem der Ansprüche 2 bis 8, **dadurch gekennzeichnet, dass** die Vielzahl von Stimulationsmitteln (121-125) einen durchbrochenen Käfig umfasst, der Abmessungen aufweist, die zur Aufnahme einer weiteren Maus geeignet sind, und der dazu eingerichtet ist, der wenigstens einen im Haus (111-115) befindlichen Maus zu ermöglichen, die in dem Käfig befindliche weitere Maus zu berühren; und dass die Vielzahl von Sensoren (131-135) eine Kamera zum Messen ihrer Interaktion umfasst

10. Vorrichtung (100) nach einem der Ansprüche 2 bis 9, **dadurch gekennzeichnet, dass** die Vielzahl von Sensoren (131-135) wenigstens einen Sensor (210) zum Erfassen und Messen der Bewegungen des wenigstens einen Maus im Labyrinth umfasst.

11. Vorrichtung (100) nach dem vorhergehenden Anspruch, **dadurch gekennzeichnet, dass** wenigstens ein Teil des Bodens (230) der Vorrichtung (100) aus einem für Infrarotstrahlung transparenten Material besteht.

12. Vorrichtung (100) nach dem vorhergehenden Anspruch, **dadurch gekennzeichnet, dass** sie weiterhin umfasst::

  - wenigstens eine Infrarotquelle (221 - 224), die auf einer ersten Seite der Vorrichtung (100) angeordnet ist und dazu eingerichtet ist, den Boden (230) der Vorrichtung (100) gleichmäßig zu beleuchten,
  - wenigstens eine infrarotstrahlungsempfindliche Kamera (210), die sich auf einer zweiten Seite der Vorrichtung (100) befindet und dazu eingerichtet ist, die Bewegungen der wenigstens einen Maus im Labyrinth dadurch zu messen, dass ihr Schatten auf dem Boden der Vorrichtung (100) erfasst wird.

13. Verfahren zum Bewerten der Orientierungsfähigkeit wenigstens einer Maus in der Vorrichtung (100) nach einem der Ansprüche 10 bis 12, wobei das Verfahren umfasst:

  - wenigstens einen Lernschritt, der darin besteht, die wenigstens eine Maus in der Vorrichtung (100) während einer vorbestimmten sogenannten Lernzeitdauer in eine Situation zu versetzen,
  - einen Schritt eines Wasser- und/oder Futterentzugs der wenigstens einen Maus während einer sogenannten Entzugsperiode,
  - einen Testschritt, während dessen die wenigstens eine Maus wieder in die Vorrichtung (100) eingebracht wird,

  die Verarbeitungseinheit bestimmt die Anzahl von Fehlern, die auf dem Weg zu jedem Haus (111-115) gemacht werden, und/oder die Laufzeit, um jedes Haus (111-115) zu betreten, und/oder den gelaufenen Weg.

**Claims**

1. A device (100) for studying the topographical memory of at least one mouse, said device (100) comprising:

- a central space, called the agora (150), open on at least one side,
- a plurality of closed spaces, called houses (111-115), located at the periphery of the agora (150), said houses (111-115) being open on one side and connected to at least one traffic space, called a street (161-165, 171-175, 181-185), allowing the at least one mouse to reach the agora (150), and
- a plurality of means of stimulation (121-125) of the at least one mouse located in the plurality of houses (111-115),

**characterized in that** said at least one traffic space (161-165, 171-175, 181-185) form a maze that is arranged in such a way that the distances between the agora (150) and each house (111-115) are identical, each house (111-115) being accessible from the agora (150) via at least two direct paths of the same length, all of the direct paths connecting each house (111-115) to the agora (150) being of equal length.

2. The device (100) according to the preceding claim, **characterized in that** it further comprises:

- a plurality of sensors (131-135) arranged for measuring the activity of the at least one mouse with the plurality of means of stimulation (121-125), and
- a processing unit arranged for determining behavioural parameters of the at least one mouse in said device (100) from measurements made by the plurality of sensors (131-135).

3. The device (100) according to the preceding claim, **characterized in that** the plurality of means of stimulation (121-125) comprises a food distributor; and **in that** the plurality of sensors (131-135) comprises at least one sensor arranged for measuring the volume of food eaten by the at least one mouse and/or the number of usages of said food distributor and/or the time spent using it.

4. The device (100) according to the preceding claim, **characterized in that** the food distributor comprises:

- a main reservoir for food,
- a feeding reservoir connected to the main reservoir, and
- a flap located between the main reservoir and the feeding reservoir, said flap being arranged to open and close automatically depending on the level of food present in the feeding reservoir.

5. The device (100) according to any one of claims 2 to 4, **characterized in that** the plurality of means of stimulation (121-125) comprises at least one drink distributor; and **in that** the plurality of sensors (131-135) comprises at least one sensor arranged for measuring the volume of liquid drunk by the at least one mouse and/or the number of times each drink distributor is used and/or the time spent using it.

6. The device (100) according to the preceding claim, **characterized in that** the drink distributor comprises:

- at least one reservoir containing a liquid, and
- a teat located at the base of said reservoir.

7. The device (100) according to any one of claims 2 to 6, **characterized in that** the plurality of means of stimulation (121-125) comprises a games platform comprising at least one tube arranged so that the at least one mouse can pass through it; and **in that** the plurality of sensors (131-135) comprises a camera for measuring the activity of the at least one mouse in the at least one tube.

8. The device (100) according to any one of claims 2 to 7, **characterized in that** the plurality of means of stimulation (121-125) comprises a running platform comprising at least one activity wheel; and **in that** the plurality of sensors (131-135) comprises at least one sensor for measuring the number of revolutions of the wheel carried out and/or the average speed and/or the instantaneous speed and/or the usage time of each activity wheel.

9. The device (100) according to any one of claims 2 to 8, **characterized in that** the plurality of means of stimulation (121-125) comprises an openwork cage of dimensions suitable for receiving another mouse and arranged to allow the at least one mouse located in the house (111-115) to touch the other mouse located in said cage; and **in that** the plurality of sensors (131-135) comprises a camera for measuring their interaction.

10. The device (100) according to any one of claims 2 to 9, **characterized in that** the plurality of sensors (131-135) comprises at least one sensor (210) for detecting and measuring the movements of the at least one mouse inside the maze.

11. The device (100) according to the preceding claim, **characterized in that** at least a part of the floor (230) of said device (100) is made of a material that is transparent to infrared radiation.

12. The device (100) according to the preceding claim, **characterized in that** it further comprises:

   - at least one infrared source (221-224) located on a first side of the device (100) and arranged for uniformly illuminating the floor (230) of said device (100),
   - at least one camera (210) sensitive to infrared radiation located on a second side of the device (100) and arranged for measuring the movements of the at least one mouse in the maze by detecting its shadow on the floor of said device (100).

13. A method for evaluating the ability of at least one mouse to orientate itself in the device (100) according to any one of claims 10 to 12, said method comprising:

   - at least one learning step consisting of leaving said at least one mouse in the device (100) for a predetermined time called the learning time,
   - a step of depriving said at least one mouse of water and/or of food for a period of time called the deprivation time,
   - a testing step, during which the at least one mouse is put back into the device (100),

   the processing unit determines the number of errors made on the path to each house (111-115), and/or the travel time for entering each house (111-115), and/or the path taken.

Figure 1

Figure 2

Figure 3

Figure 4

Figure 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 101884306 A
- US 20130233249 A

- CN 201995441 U

**Littérature non-brevet citée dans la description**

- **G. WINOCUR et al.** *Neurobiology of Aging,* 2010, vol. 31, 143-150